Europäisches Patentamt

European Patent Office

Office européen des brevets

(11) Publication number: **0 070 906**
**B1**

# EUROPEAN PATENT SPECIFICATION

(45) Date of publication of patent specification: **15.10.86**

(21) Application number: **82900385.4**

(22) Date of filing: **04.02.82**

(86) International application number:
**PCT/JP82/00034**

(87) International publication number:
**WO 82/02715 19.08.82 Gazette 82/20**

(51) Int. Cl.⁴: **C 07 H 21/04, C 12 N 15/00** // **C12P19/34, C12P21/00**

(54) **HUMAN INTERFERON-BETA GENE.**

(30) Priority: **04.02.81 JP 14373/81**
**11.07.81 JP 108539/81**

(43) Date of publication of application:
**09.02.83 Bulletin 83/06**

(45) Publication of the grant of the patent:
**15.10.86 Bulletin 86/42**

(84) Designated Contracting States:
**DE FR GB**

(56) References cited:
**JP-A-57 024 400**

**NUCLEIC ACIDS RESEARCH, vol. 9, no. 2, 1981 M. HOUGHTON et al.: "The absence of introns within a human fibrolabst interferon gene", pages 247-266**

**NUCLEIC ACIDS RESEARCH, vol. 9, no. 5, 1981 R.M. LAWN et al.: "Human fibroblast interferon gene lacks introns", pages 1045-1053**

**PROC. NATL. ACAD. SCI. USA, vol. 78, no. 9, September 1981 S. OHNO et al.: "Structure of a chromosomal gene for human interferon bèta", pages 5305-4309**

(73) Proprietor: **JURIDICAL FOUNDATION JAPANESE FOUNDATION FOR CANCER RESEARCH**
**37-1, Kamiikebukuro 1-chome Toshima-ku Tokyo 170 (JP)**

(72) Inventor: **SUGANO, Haruo**
**4-8-13, Minamiogikubo Suginami-ku Tokyo 167 (JP)**
Inventor: **TANIGUCHI, Tadatsugu**
**Yuparesu Tagara 303 4-27-12, Tagara, Nerima-ku Tokyo 176 (JP)**
Inventor: **ONO, Shigeo**
**4-4-8, Yakumo, Meguro-ku Tokyo 152 (JP)**

(74) Representative: **Vossius & Partner Siebertstrasse 4 P.O. Box 86 07 67 D-8000 München 86 (DE)**

(56) References cited:
**NUCLEIC ACIDS RESEARCH, vol. 10, no. 3, 1982 S. OHNO et al.: "Inducer-responsive expression of the cloned human interferon bètal gene introduced into cultured mouse cells", pages 967-977**

Courier Press, Leamington Spa, England.

(56) References cited:

EUR. J. BIOCHEM. vol. 120, 1981, Y. MORI et al.: "Synthesis of human interferon bètal in Escherichia coli infected by a lambda phage recombinant containing a human genomic fragment", pages 197-202

Nature, Vol. 285, No. 19 (June, 1980) P542 - 549. Especially see 547-549

Ishikawa Kunihiko Henshu "(Bessatsu Tanpakushitsu Kakusan Koso) Interferon Kenkyu no Shinpo" 1. December. 1981 (01.12.81) Kyoritsu Shuppan Kabushiki Kaisha P169 - 182, Especially see page 174 to 175

## Description

A DNA fragment which is isolated from human chromosomal DNA essentially consisting of the gene coding for human interferon-β protein and a DNA sequence responsible for regulating the transcription of the gene in eukaryotes, the recombinant vector and the use of the recombinant vector.

The present invention relates to a DNA fragment which is isolated from human chromosomal DNA essentially consisting of the gene coding for human interferon-β and a DNA sequence responsible for regulating the transcription of said gene in eukaryotes, a recombinant vector DNA containing said DNA fragment and the use of said recombinant vector DNA in a process for preparing interferon-β protein.

It has been known that a cDNA of human interferon-β is prepared using mRNA as a template; see T. Taniguchi et al., Gene 10, 11—15, (1980).

The present inventors have studied the mass production of interferon-β using a recombinant DNA wherein the human interferon-β gene is inserted into a plasmid DNA, for example, a plasmid DNA derived from Escherichia coli or a phage DNA, for example, lambda phage DNA derived from Escherichia coli lambda phage by recombinant DNA technology. As a result, it has been found that human interferon-β can be produced by propagating and multiplying a novel recombinant vector DNA in bacteria such as Escherichia coli and a compound having the same structure as human interferon-β can also be produced by propagating the recombinant vector DNA in eukaryotic cells such as mouse cells either in the autoreplicative form or by integration into the host chromosomes.

The recombinant vector DNA of the present invention is novel and it contains at least the entire region coding for the chromosomal human interferon-β gene and furthermore it contains a region which is believed to be responsible for the control of transcription in eukaryotes.

According to the present invention a DNA fragment from chromosomal human DNA is obtained, which essentially consists of gene coding for human interferon-β protein and a DNA sequence responsible for regulating the transcription of this gene in eukaryotes.

Furthermore the present invention provides a recombinant vector DNA containing said DNA fragment and a vector DNA region.

The recombinant vector DNA of the present invention is prepared by the following procedure.

Total human chromosomal DNA is extracted from human fetal liver and is cut with a restriction endonuclease into fragments varying in length.

Fragments having a certain length and containing the interferon-β gene are isolated by preparative agarose gel electrophoresis. The isolated fragments are inserted into a vector DNA by recombinant DNA technology to obtain recombinant vector DNA molecules. The novel recombinant vector DNA containing the chromosomal human interferon-β gene is selected and isolated from all recombinant DNA molecules using as a probe radioactively labelled complementary DNA (cDNA) prepared from human interferon-β messenger RNA.

The process for producing said recombinant vector DNA is specifically explained in more detail.

Human chromosomal DNA is prepared from human fetal liver by phenol extraction. The extracted DNA is subjected to partial digestion with restriction endonucleases such as HaeIII and AluI.

The thus obtained entire DNA fragments of human chromosomal DNA are joined with EcoRI linker, and inserted into a DNA such as that of lambda phage of Escherichia coli, using bacteriophage T4 ligase to obtain recombinant DNAs. The recombinant DNAs are packaged in vitro into lambda phage heads in order to utilize the efficiency of phage infection [Lawn et al., Cell 15, 1157—1174 (1978)] which is considerably higher than transformation or transfection of cells by pure DNA. The thus obtained whole recombinant DNAs are called human genomic library. The human genomic library contains almost all human genomic DNAs as apparent from the construction method and most of the human genes can be isolated therefrom.

A recombinant DNA having a DNA fragment containing the human interferon-β gene can be selected from the thus obtained human genomic library as follows.

A recombinant plasmid having a DNA insert which is complementary to human interferon-β messenger RNA (cDNA) is propagated in Escherichia coli ×1776/TpIF319—13 ATCC 31712 and isolated according to the method of Currier and Nester [Analyt. Biochem. Vol. 76, 431—441 (1976)]. The plasmid DNA is labelled with [$^{32}$P] by nick translation as described by Roop et al., Cell 15, 671—685 (1978) and used as a probe.

The genomic library prepared as mentioned above by using Escherichia coli lambda phage as a vector is dispersed in appropriate dilution together with E. coli cells as indicator on an agar plate. After incubation phage plaques appear, each representing a clone. DNA in the phage plaques is transferred to and fixed on a nitrocellulose filter according to the method of Benton and Davis, Science, 196, 180—182 (1977).

The filter is subjected to hybridization using the probe mentioned above. Recombinant phage DNA hybridizing with this probe is detected by autoradiography. The phages from the corresponding plaques are isolated from the agar plate.

The thus obtained phage stock is propagated in E. coli and DNA is extracted therefrom. The DNA is treated with a restriction endonuclease such as EcoRI and the resulting fragments are separated by agarose gel electrophoresis. The separated DNA fragments are transferred and fixed on a nitrocellulose filter as described by E. Southern, J.

Mol. Biol. 98, 503—517 (1975). Hybridization is carried out using the probe described above.

Thus, a phage clone including an EcoRI fragment of 1.8 kb which hybridizes with the cDNA is obtained.

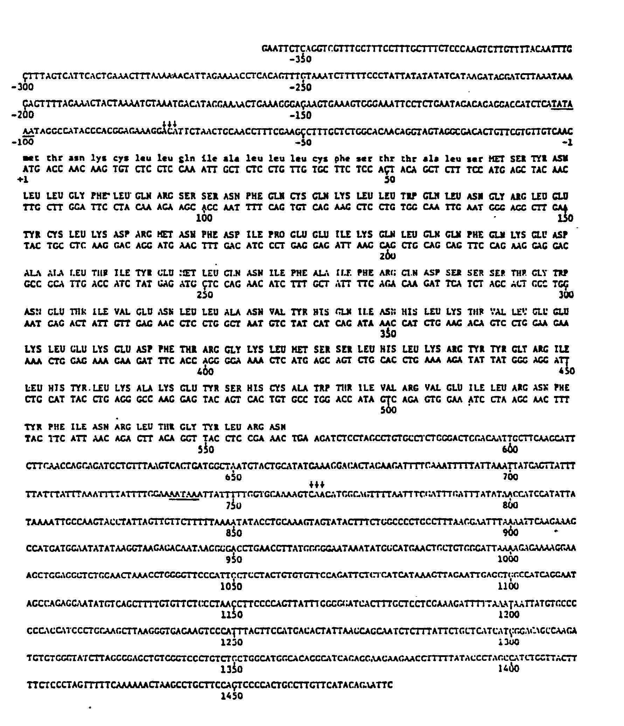

A more detailed restriction endonuclease map is prepared by the method of Smith and Birnstiel, Nucleic Acids Res. 3, 2387—2398 (1976); see Fig. 2.

Further, the DNA base sequence is determined as described by Maxam and Gilbert, Proc. Natl. Acad. Sci. USA 74. 560—564 (1977). The DNA base sequence is compared with that of the human interferon-β cDNA [T. Taniguchi et al., Gene 10, 11—15 (1980)] whereby the clone obtained is confirmed to have a chromosomal gene corresponding to human interferon-β messenger RNA, that is, chromosomal human interferon-β gene.

Brief description of the drawing

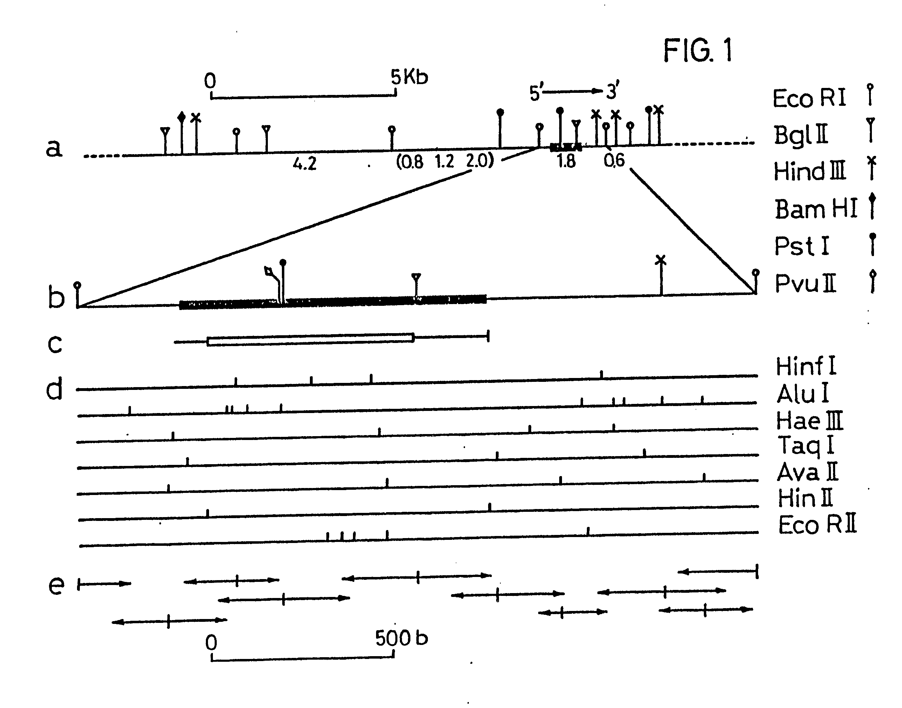

Figure 1-a shows a restriction endonuclease map of the 15 kb chromosomal DNA fragment cloned in λHIFN-β₁-121. The broken line in the figure indicates the arms of the vector DNA derived from Charon 4A.

Figures 1-b and 1-d show a restriction endonuclease map of 1.8 kb EcoRI fragment derived from human chromosomal DNA. The black box shows the region from which the messenger RNA is transcribed.

Figure 1-c shows a region in human chromosomal DNA corresponding to interferon-β cDNA. The open box in the figure indicates a protein coding region.

Figure 1-e shows the strategy for sequence determination. Arrows in the figure indicate the direction and extent of the sequence of each fragment analysed.

Figure 2 shows the base sequence of the 1.8 kb EcoRI fragment. The section numbered +1 to +561 indicates the sequence coding for the human interferon-β protein, the arrows at −73 to −75 indicate the initiation site for transcription, the TATA box is underlined.

A specific embodiment of the present invention is explained in the example.

Example 1

I. Preparation of a human genomic library

The human genomic library prepared by the following method was provided by Tom Maniatis (California Institute of Technology).

Total human chromosomal DNA is extracted from human fetal liver with phenol and subjected to partial digestion with restriction endonucleases HaeIII and AluI.

Fragments of about 18—25 kb length are isolated by sucrose density gradient centrifugation. Then, the isolated fragments are connected to EcoRI linkers which are then ligated to the EcoRI fragments (19 kb and 11 kb) of lambda phage Charon 4A. Then, the recombinant DNA is packaged in vitro to give complete and infectious lambda phage particles. The resultant human genomic library is in principle believed to be a population of recombinant DNAs containing 18—25 kb human DNAs containing almost all human genes.

II. Preparation of a radioactive probe representing the human interferon-β-cDNA

The recombinant phage having a DNA fragment containing the human interferon-β gene was selected from the human genomic library using as a probe a [³²P]-labelled cDNA fragment having the entire cDNA region translatable to human interferon-β protein by the method of Benton and Davis, Science 196, 180—182 (1977). Details are shown below.

A DNA fragment of about 0.57 kb having the cDNA region translatable to human interferon-β protein and being used as a probe was prepared and radio-labelled as follows.

TpIF319-13 plasmid DNA is isolated from Escherichia coli ×1776/TpIF319-13 ATCC 31712 carrying the recombinant plasmid TpIF319-13 containing the human interferon-β cDNA by the method of Currier and Nester, Analyt. Biochem. 76, 431—441 (1976), and digested with restriction endonucleases HincII, BglII and HhaI. The longest DNA fragment in the digest, i.e. a 0.57 kb DNA, is the desired DNA fragment which is isolated by agarose gel electrophoresis according to the method of Tabak and Flavell, Nucleic Acids Research 5, 2321—2332 (1978).

Ths DNA fragment is radio-labelled with [³²P] by nick translation as described by Roop et al., Cell 15, 671—685 (1978).

III. Selection and identification of a lambda phage clone containing human interferon-β-gene

This probe is used for the identification of recombinant lambda phage DNA containing the chromosomal human interferon-β gene.

For clonal distribution the λphage particles together with E. coli-indicator cells are dispersed on an agar plate [Science 202, 1279—1284 (1978)] to form phage plaques. The density of phage plaques is 10,000 to 30,000 per plate with a diameter of 15 cm.

A nitrocellulose filter (Schleicher and Schüll) is layered on the agar plate and marked by inkspots for correct orientation of the filter relative to the plate and for identifying later, aided by the autoradiogram, the positive plaques. The agar plate is allowed to stand at 4°C for about 20 minutes to adsorb phages on the filter. The plate is kept at 4°C and the nitrocellulose filter is air-dried at room temperature for about 90 minutes. It is soaked in an aqueous solution consisting of 0.1N NaOH and 1.5M NaCl for about 20 seconds to denature the phage DNAs. Then, they are neutralized in a solution of 0.2M Tris-HCl (pH 7.4) and 2×SSC (SSC means an aqueous solution consisting of 0.15M NaCl and 0.015M sodium citrate and 2×SSC means 2-fold concentrated SSC) for about 20 seconds and additionally in 2×SSC for about 20 seconds. After air-drying at room temperature for one hour and at 80°C for 3 hours, the denatured phage DNAs are fixed on the nitrocellulose filter.

Hybridization of the denatured phage DNA on the nitrocellulose filter is carried out using the radiolabelled human interferon-β cDNA as a probe as follows.

The nitrocellulose filter is incubated in 3×SSC at 65°C for 30 minutes and in a 3×SSC solution containing 0.2% polyvinylpyrrolidone (product of Nakarai Kagaku Co.), 0.2% bovine serum albumin (product of Iwai Kagaku Co.) and 0.2% Ficoll (Product of Pharmacia Fine Chemicals) at 65°C for 60 minutes. Then the paper is incubated at 65°C in a hybridization solution consisting of 1M NaCl, 50 mM Tris-HCl (pH 8.0), 10 mM EDTA, 0.1% SDS, and 100 μg/ml ultrasonicated and heat-denatured Escherichia coli DNA for 60 minutes, whereby the whole hybridization treatment is completed.

Separately, the ratio-labelled probe DNA is denatured by heating at 95°C for 10 minutes. Then, the pretreated nitrocellulose filter and the heat-denatured probe DNA are incubated in the hybridization solution mentioned above at 65°C for hybridization. After 12 to 18 hours, the nitrocellulose filter is picked up, washed with 2×SSC twice, incubated at 65°C in a solution containing 0.3×SSC and 0.1% SDS for 60 minutes twice, air-dried at 80°C for one hour and subjected to radioautography using an X-ray film.

For identification and isolation of recombinant lambda phages from the plate which was kept at 4°C in the meantime, the autoradiogram and the plate are superimposed in the correct orientation (given by the inkspots, supra). Recombinant lambda phages which hybridized with the probe are scraped off with toothpicks. The procedure for identification and isolation is repeated in order to obtain a pure clone of the identified recombinant phage.

Thus, 11 clones are obtained by screening about 100 million phage plaques.

IV. Southern hybridization

The recombinant DNA of each clone is prepared by the method of Maniatis [Cell 15, 687—701 (1978)] and used in the following analysis.

The recombinant DNA of each clone is cleaved with the restriction endonuclease EcoRI and chain lengths of DNA fragments formed are determined by agarose gel electrophoresis and ethidium bromide staining. The DNA digests of all clones have 19 kb and 11 kb DNA fragments derived from the arms of the cloning vector lambda phage Charon 4A as well as some DNA fragments derived from human chromosomal DNA. By this analysis, 11 clones are classified to 5 species. Further, the Southern hybridization [Southern, J. Mol. Biol. 98, 503—517 (1975)] is carried out using the human interferon-β cDNA probe employed in the screening mentioned above to determine which DNA fragments obtained by the digestion with EcoRI hybridize with human interferon cDNA.

That is, the DNA in each phage clone is digested with EcoRI and subjected to agarose gel electrophoresis. Thereafter, gels are recovered by cutting and incubated at room temperature in an aqueous solution containing 0.5N NaOH and 1M NaCl to denature the DNA. The same procedure is repeated in an aqueous solution containing 0.5N Tris-HCl (pH 7.0) and 1.5M NaCl to neutralise the gel. The gel is placed on a filter paper soaked with 20×SSC and covered with a nitrocellulose filter. Then, a filter paper and a staple of paper are put on the nitrocellulose filter to soak the denatured DNA into the nitrocellulose filter. After 12 to 18 hours, the nitrocellulose filter is peeled off from the gel and baked at 80°C for 3 hours in vacuum to fix the DNA on the nitrocellulose filter. Hybridization is carried out in the same manner as for screening of phage DNA described above.

Thus, it is confirmed that four species of the human chromosomal gene fragments in the five species have a 1.8 kb DNA fragment generated by cleavage with the restriction endonuclease EcoRI, referred to as "EcoRI fragment" hereinafter. The 1.8 kb EcoRI fragment has a sequence complementary to human interferon-β cDNA. The other species are confirmed to have a DNA fragment containing a part of the 1.8 kb EcoRI fragment.

One of the recombinant lambda phage DNA clones containing the 1.8 kb EcoRI fragment is designated λHIFN-β$_1$-121. A cleavage map for this clone is prepared using restriction endoculceases such as HindIII, BamHI, BglII, PstI and other endonucleases mentioned in Fig. 1-a. The map is illustrated in Fig. 1-a.

In order to investigate the 1.8 kb EcoRI fragment showing complementarity to the human interferon-β cDNA, the fragment is subcloned into plasmid pBR322 as a vector as follows.

V. C-tailing of 1.8 kb EcoRI-fragment

One μg of λHIFN-β$_1$-121 DNA is digested with the restriction endonuclease EcoRI and the resulting cohesive ends are rendered flush with 5 units of DNA polymerase I Klenow fragment (product of Boehringer Mannheim) in 30 μl of an aqueous solution containing 0.1M potassium phosphate buffer solution (pH 6.9), 6 mM MgCl$_2$, 6 mM mercaptoethanol, 1 mM dATP and 1 mM dTTP. After removal of proteins with phenol, the aqueous phase is taken from which the DNA is recovered for tailing. The purified DNA is treated at 37°C in a reaction mixture of 30 μl consisting of 1 μg of DNA, 0.14 M potassium cacodylate (pH 7.6), 0.03 M Tris, 0.1 mM dithiothreitol, 1 mM CaCl$_2$, 1 mM dCTP and 2 units of terminal transferase for 15 minutes to tail the 3' ends of the EcoRI fragment wiwh about 100 deoxycytidine residues. Separately, a vector is prepared by cutting pBR322 with PstI and tailing the 3' ends of the PstI fragment with about 100 deoxyguanine residues.

IV. Subcloning of the 1.8 kb EcoRI fragment into plasmid pBR322

For subcloning 0.05 μg of the thus obtained 1.8 kb EcoRI fragment tailed with dC and 0.05 μg of pBR322 DNA tailed with dG are incubated at 65°C for 2 hours, at 45°C for one hour, at 37°C for one hour and at room temperature for one hour in a solution consisting of 0.1M NaCl, 50 mM Tris-HCl (pH 7.5), and 5 mM EDTA for annealing the oligo dC and oligo dG ends. Escherichia coli ×1776 is subjected to transformation with the reaction mixture by the method of Enea et al., J. Mol. Biol. 96, 495—509 (1975). Tetracycline resistant strains are picked and the DNAs of 400 bacterial clones are fixed on a nitrocellulose filter [Grunstein-Hogness method, Proc. Natl. Acad. Sci. USA 72, 3961—3965 (1975)]. Hybridization is carried out on the nitrocellulose filter in the same manner as for screening of recombinant phage DNA as described above and using the same probe of human interferon-β cDNA, with the exception that heat denatured pBR322 was added to a final concentration of 30 μg/ml, for the selection of ×1776 Escherichia coli clones having a recombinant plasmid pBR322 containing the 1.8 kb EcoRl fragment.

A recombinant plasmid pBR322 containing the 1.8 kb EcoRl fragment which hybridizes with human interferon-β cDNA is prepared from the thus obtained ×1776 Escherichia coli clone by the method of Currier and Nester mentioned above and analysed as follows.

It is apparent from the above that the 1.8 kb EcoRl fragment derived from the human chromosomal DNA contains a DNA complementary to the messenger RNA of human interferon-β DNA. For further confirmation, a cleavage map for restriction endonucleases is prepared by measuring the chain lengths of the DNA fragments generated by cutting the recombinant plasmid DNA or a part thereof with one or more restriction endonucleases or by partially digesting the fragment labelled with [$^{32}$P] at the 3' ends using polynucleotide kinase [Smith and Birnstiel, Nucleic Acids Res., 3, 2387—2398 (1976)] (see Fig. 1-b, -d) on agarose- or polyacrylamide-gels. Fig. 1-c shows the region corresponding to interferon-β cDNA wherein the open box indicates the protein coding region. The same region as the cDNA is ·found in the cleavage map. It is apparent from the foregoing that the 1.8 kb EcoRl DNA fragment derived from human chromosomal DNA has the same restriction map as the cDNA derived from human interferon-β messenger RNA, i.e. the 1.8 kb EcoRl DNA fragment of the chromosomal DNA is suspected to contain the human interferon-β gene (see below) (the black box in Fig. 1-b).

It has been shown that intervening sequences, (introns) present in the genes of many eukaryotic cells are absent in the human interferon-α gene [Nagata, S., Mantei, N., Weissmann C., Nature, 287, 401 (1980)]. The absence of intervening sequences in the interferon-β gene in the 1.8 kb EcoRl fragment suggests that the genomic DNA is useful for the production of interferon proteins by prokaryotic organisms such as Escherichia coli not having the splicing mechanism for removing introns.

In order to prove the foregoing, the base sequence of the 1.8 kb EcoRl fragment was determined according to the method of Maxam and Gilbert [Proc. Natl. Acad. Sci. USA 74, 560—564 (1977)]. The result is shown in Fig. 2. The nucleotide sequence of the coding region of this cloned gene was identical to that of the cDNA of TpIF319-13 which had been cloned previously. The 1.8 kb EcoRl fragment introduced into Escherichia coli has been deposited with the American Type Culture Collection in U.S.A. as Escherichia coli Cl4 under accession number ATCC 131905.

## Claims

1. A DNA fragment which is isolated from human chromosomal DNA essentially consisting of the gene coding for human interferon-β protein and a DNA sequence responsible for regulating the transcription of the gene in eukaryotes.

2. A recombinant vector DNA containing a DNA fragment according to claim 1.

3. The recombinant vector DNA according to claim 2, characterized in that the vector DNA is selected from lambda phage, Charon phages and plasmid pBR322, pCR1, pMB9 and pSC1 derived from Escherichia coli.

4. The use of the recombinant vector DNA according to claim 2 or 3 in a process for preparing interferon-β protein by prokaryotes or eukaryotes.

## Patentansprüche

1. Aus menschlicher chromosomaler DNA isoliertes DNA-Fragment, im wesentlichen bestehend aus dem für das menschliche β-Interferon-Protein codierenden Gen und einer für die Regulation der Transkription des Gens in Eukaryonten verantwortlichen DNA-Sequenz.

2. Rekombinante Vektor-DNA, enthaltend ein DNA-Fragment nach Anspruch 1.

3. Rekombinante Vektor-DNA nach Anspruch 2, dadurch gekennzeichnet, daß die Vektor-DNA vom Lambda-Phagen, von Charon-Phagen oder von den Escherichia coli-Plasmiden pBR322, pCR1, pMB9 oder pSC1 stammt.

4. Verwendung der rekombinanten Vektor-DNA nach Anspruch 2 oder 3 in einem Verfahren zur Herstellung des β-Interferon-Proteins durch Prokaryonten oder Eukaryonten.

## Revendications

1. Fragment d'ADN qui est isolé d'un ADN de chromosome humain, essentiellement constitué du gène codant pour la protéine interféron-β humain et d'une séquence d'ADN responsable du contrôle de la transcription du gène dans les eucaryotes.

2. Recombinant d'ADN vecteur contenant un fragment d'ADN conforme à la revendication 1.

3. Recombinant d'ADN vecteur conforme à la revendication 2, caractérisé en ce que l'ADN vecteur est choisi parmi le phage lambda, les phages Charon et les plasmides pBR322, pCR1, pMB9 et pSC1 obtenus à partir d'Escherichia coli.

4. Utilisation du recombinant d'ADN vecteur conforme à la revendication 2 ou 3 dans un procédé de préparation de la protéine interféron-β par des procaryotes ou des eucaryotes.

FIG. 1

```
                                          GAATTCTCACGTCGTTTGCTTTCCTTTGCTTTCTCCCAAGTCTTGTTTTACAATTTG
                                          -350
CTTTAGTCATTCACTGAAACTTTAAAAAACATTAGAAAACCTCACAGTTTGTAAATCTTTTTCCCTATTATATATATCATAAGATACGATCTTAAATAAA
-300                                       -250
GAGTTTTAGAAACTACTAAAATGTAAATGACATAGGAAAACTGAAAGGGACAAGTGAAAGTCGGAAATTCCTCTGAATACACACAGCACCATCTCATATA
-200                                       -150
AATAGGCCATACCCACGGAGAAAGGACATTCTAACTGCAACCTTTCGAAGCCTTTGCTCTGGCACAACAGGTAGTAGGCGACACTGTTCGTGTTGTCAAC
-100                                       -50                                       -1

met thr asn lys cys leu leu gln ile ala leu leu leu cys phe ser thr thr ala leu ser MET SER TYR ASN
ATG ACC AAC AAG TGT CTC CTC CAA ATT GCT CTC CTG TTG TGC TTC TCC ACT ACA GCT CTT TCC ATG AGC TAC AAC
+1                                                      50

LEU LEU GLY PHE LEU GLN ARG SER SER ASN PHE GLN CYS GLN LYS LEU LEU TRP GLN LEU ASN GLY ARG LEU GLU
TTG CTT GGA TTC CTA CAA ACA AGC ACC AAT TTT CAG TGT CAG AAG CTC CTG TGC CAA TTG AAT GGG AGG CTT GAA
                           100                                                       150

TYR CYS LEU LYS ASP ARG MET ASN PHE ASP ILE PRO GLU GLU ILE LYS GLN LEU GLN GLN PHE GLN LYS GLU ASP
TAC TGC CTC AAG GAC ACG ATG AAC TTT GAC ATC CCT GAG GAG ATT AAG CAG CTG CAG CAG TTC CAG AAG GAG GAC
                                                       200

ALA ALA LEU THR ILE TYR GLU MET LEU GLN ASN ILE PHE ALA ILE PHE ARG GLN ASP SER SER SER THR GLY TRP
GCC GCA TTG ACC ATC TAT GAG ATG CTC CAG AAC ATC TTT GCT ATT TTC AGA CAA GAT TCA TCT ACC ACT GCC TGG
                           250                                                       300

ASN GLU THR ILE VAL GLU ASN LEU LEU ALA ASN VAL TYR HIS GLN ILE ASN HIS LEU LYS THR VAL LEU GLU GLU
AAT CAG ACT ATT GTT GAG AAC CTC CTG GCT AAT GTC TAT CAT CAG ATA AAC CAT CTG AAG ACA GTC CTG GAA GAA
                                                       350

LYS LEU GLU LYS GLU ASP PHE THR ARG GLY LYS LEU MET SER SER LEU HIS LEU LYS ARG TYR TYR GLY ARG ILE
AAA CTG GAG AAA GAA GAT TTC ACC AGG GGA AAA CTC ATG AGC AGT CTG CAC CTG AAA AGA TAT TAT GGC AGG ATT
                           400                                                       450

LEU HIS TYR LEU LYS ALA LYS GLU TYR SER HIS CYS ALA TRP THR ILE VAL ARG VAL GLU ILE LEU ARG ASN PHE
CTG CAT TAC CTG ACG GCC AAG GAG TAC AGT CAC TGT GCC TGG ACC ATA GTC AGA GTG GAA ATC CTA AGC AAC TTT
                                                       500

TYR PHE ILE ASN ARG LEU THR GLY TYR LEU ARG ASN
TAC TTC ATT AAC AGA CTT ACA GGT TAC CTC CGA AAC TGA ACATCTCCTAGCCTGTGCCTCTCGGACTCGACAATTGCTTCAAGCATT
                           550                                                       600

CTTCAACCAGCAGATCCTGTTTAAGTCACTGATGGCTAATGTACTGCATATCAAAGGACACTACAAGATTTTCAAATTTTTATTAAATTATCAGTTATTT
                           650                                                       700
                                                      +++
TTATTTATTTAAATTTTATTTTGGAAAATAAATTATTTTTTGGTGCAAAAGTCAACATGGCAGTTTTTAATTTCGATTTGATTTATATAACCATCCATATTA
                           750                                                       800

TAAAATTGCCAAGTACCTATTAGTTGTTCTTTTTTAAAATATACCTGCAAAGTAGTATACTTTCTGGCCCCTGCCTTTAAGGAATTTAAAATTCAAGAAAG
                           850                                                       900

CCATGATGGAATATATAAGGTAAGACACAATAAGGGGCACCTGAACCTTATGGGGGAATAAATATGGCATGAACTGCTGTCGGGATTAAAAGAGAAAGGAA
                           950                                                       1000

AGCTGGAGGGTCTGCAACTAAACCTGGGGTTCCCATTCCTCCTACTGTGTGTTCCAGATTCTCTCATCATAAAGTTAGAATTGAGGTGGCCATCAGGAAT
                           1050                                                      1100

AGCCAGAGCAATATGTCAGCTTTTGTGTTCTCCCTAACCTTCCCCAGTTATTTGGGGGATCACTTTGCTCCTCCAAAGATTTTTAAATAATTATGTGCCC
                           1150                                                      1200

CCCACCATCCCTGCAAGCTTAAGGGTGACAAGTCCCATTTACTTCCATGACACTATTAAGCAGCAATCTCTTTATTCTGCTCATCATGGGACAGCCAAGA
                           1250                                                      1300

TGTCTGGGTATCTTAGCGGAGCTGTCGGTCCCTGTCTCCTGGCATGGCACACGGCATCAGAGCAAGAAGAACCTTTTTATACCCTAGCCATCTCGTTACTT
                           1350                                                      1400

TTCTCCCTAGTTTTTCAAAAAACTAAGCCTGCTTCCAGTCCCCACTGCCTTGTTCATACAGAATTC
                           1450
```